(19) Europäisches Patentamt
European Patent Office
Office européen des brevets

(11) EP 2 727 999 B1

(12) **EUROPEAN PATENT SPECIFICATION**

(45) Date of publication and mention of the grant of the patent:
**29.03.2017 Bulletin 2017/13**

(51) Int Cl.:
***C12N 11/00*** *(2006.01)* ***C12N 11/14*** *(2006.01)*

(21) Application number: **12192344.5**

(22) Date of filing: **13.11.2012**

(54) **IMMOBILIZED ENZYME AND ITS FABRICATION METHOD AND REACTION SYSTEM**

IMMOBILISIERTES ENZYM UND DESSEN HERSTELLUNGSVERFAHREN UND REAKTIONSSYSTEM

ENZYME IMMOBILISÉE ET SON PROCÉDÉ DE FABRICATION ET SYSTÈME DE RÉACTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.11.2012 TW 101140501**

(43) Date of publication of application:
**07.05.2014 Bulletin 2014/19**

(73) Proprietor: **Chung Yuan Christian University**
**Tao-Yuan (TW)**

(72) Inventors:
- **CHENG, Chean-Yeh**
**Tao-Yuan (TW)**
- **CHANG, Kuo-Chung**
**Tao-Yuan (TW)**

(74) Representative: **Karakatsanis, Georgios**
**Haft Karakatsanis Patentanwaltskanzlei**
**Dietlindenstrasse 18**
**80802 München (DE)**

(56) References cited:
**WO-A2-2004/065928 KR-A- 20130 046 205**

- **RENEE HAN-YI CHANG ET AL: "Cellulase immobilized mesoporous silica nanocatalysts for efficient cellulose-to-glucose conversion", GREEN CHEMISTRY, vol. 13, no. 10, 1 January 2011 (2011-01-01), page 2844, XP055049355, ISSN: 1463-9262, DOI: 10.1039/c1gc15563f**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 727 999 B1

## Description

## BACKGROUND OF THE INVENTION

### 1. FIELD OF THE INVENTION

The present invention relates to immobilized enzymes, formation methods thereof, and reaction systems using the immobilized enzymes.

### 2. DESCRIPTION OF THE PRIOR ART

Since the limited amount of fossil fuels and environmental concerns, there is an urgent need to develop sustainable green energy resources to solve the problems associated with the great demand of energy and the environmental protection. Renewable waste natural materials such as dead tree branch, rice straw, corn stover, bagasse, and perennial grass enrich lignocellulosic polysaccharides which can be hydrolyzed to produce glucose and subsequently fermented to obtain bioethanol for alternative energy production[19]. Presently, the thermal acid hydrolysis[21] or enzymatic hydrolysis[4-8, 25], are the two most common methods used for converting these renewable biomass to carbohydrates and further to biofuel. However, the depolymerization of lignocellulosic biomass by thermal acid hydrolysis usually exhibits by-products formation and adverse reaction conditions which make it unfavorable to an efficient bioenergy production and is environmental unfriendly. In contrast to the thermal acid hydrolysis, enzymatic hydrolysis shows almost no by-product formation and the conditions used for reaction are usually mild. However, the high cost of enzyme makes the production of biofuel impractical for large-scale industrial applications.

In order to reduce the cost of the enzymatic hydrolysis, the strategy usually used to reach this goal is the repeated use of enzyme through enzyme immobilization[11,20] that is through the immobilization of enzyme on a solid support, the homogeneous enzyme can become a heterogeneous enzyme, thus the enzyme can be recovered and recycled easily and used again and again for the hydrolysis[14]. Literature shows many enzyme immobilization methods, including the sol-gel encapsulation[25, 5, 8], adsorption[23], cross-linking[15, 17-18], chemical covalent bonding[3, 9, 14], and so on. However, in terms of long-term reusable enzyme, enzyme immobilization by chemical bonding seems the most effective one. In the past years, various solid supports had been utilized for the enzyme immobilization, for instances, Li et al. immobilized the cellulase onto the outer membrane of liposomes[18], Wu et al. attached the cellulase to the poly (vinyl alcohol)[26], and Tébéka et al. used the Si wafer as the substrate for cellulase immobilization[23]. Others materials such as polystyrene[19], polyurethane foam[2], chitin and nylon[24], and silica[3, 9, 13, 17] were also used for cellulase immobilization. However, none of these immobilization methods demonstrated a long-term and efficient reusability of cellulase for the hydrolysis of lignocellulosic materials to produce glucose.

References of the present invention are listed as follows: **(1)** Brown, K.R., Walter, D.G., Natan, M.J., 2000. Seeding of colloidal Au nanoparticle solutions. 2. improved control of particle size and shape. Chem. Mater. 12, 306-313; **(2)** Chakrabarti, A. C., Storey, K. B., 1988. Immobilization of cellulase using polyurethane foam. Appl. Biochem. Biotechnol. 19, 189-207; **(3)** Chang, R. H.-Y., Jang, J., Wu, K. C.-W., 2011. Cellulase immobilized mesoporous silica nanocatalysts for efficient cellulose-to-glucose conversion. Green Chem. 13, 2844-2850; **(4)** Cheng, C., 1998. Cellulase activity in different buffering media during waste paper hydrolysis by HPLC. J. Chin. Chem. Soc. 45, 679-688; **(5)** Cheng, C., Chang, K.-C., 2007. Sampling, dilution, and loading device-coupled high-performance liquid chromatography method for successive on-line analyses of major carbohydrate products in immobilized cellulase hydrolysate of paper cellulose. Anal. Sci. 23, 305-310; **(6)** Cheng, C., Chang, K.-C., Chen, C.-S., Pijanowska, D. G., 2011. Biosensor with nano-gold particle modified pencil lead carbon electrode for long-term glucose monitoring of waste tree branch hydrolysis. J. Chin. Chem. Soc. 58, 1-10; **(7)** Cheng, C., Chang, K.-C., Pijanowska, D. G., 2012. On-line flow injection analysis using gold particle modified carbon electrode amperometric detection for real-time determination of glucose in immobilized enzyme hydrolysate of waste bamboo chopsticks. J. Electroanal. Chem. 666, 32-41; **(8)** Cheng, C., Chen, C.-S., Hsieh, P.-H., 2010. On-line desalting and carbohydrate analysis for immobilized enzyme hydrolysis of waste cellulosic biomass by column-switching high-performance liquid chromatography. J. Chromatogr., A 1217, 2104-2110; (9) Cho, E. J., Jung, S., Kim, H. J., Lee, Y. G., Nam, K. C., Lee, H.-J., Bae, H.-J., 2012. Co-immobilization of three cellulases on Au-doped magnetic silica nanoparticles for the degradation of cellulose. Chem. Commun. 48, 886-888; **(10)** Ge, Y., Burmaa, B., Zhang, S., Wang, S., Zhou, H., Li, W., 1997. Co-immobilization of cellulase and glucose isomerase by molecular deposition technique. Biotechnol. Tech. 11, 359-361; **(11)** Geddes, C.C., Peterson, J.J., Roslander, C., Zacchi, G., Mullinix, M.T., Shanmugam, K.T., Ingram, L.O., 2010. Optimizing the saccharification of sugar cane bagasse using dilute phosphoric acid followed by fungal cellulases. Bioresour. Technol. 101, 1851-1857; **(12)** Gupta, P., Kumar, V., Paul, S., J. 2010. Silica functionalized sulfonic acid catalyzed one-pot synthesis of 4,5,8a-Triarylhex-ahydropyrimido [4,5-d]pyrimidine-2,7(1H,3H)-diones under liquid phase catalysis. Braz. Chem. Soc. 21, 349-354; **(13)** Hartono, S. B., Qiao, S. Z., Liu, J., Jack, K., Ladewig, B. P., Hao, Z., Lu, G. Q. M., 2010. Functionalized mesoporous silica with very large pores for

cellulase immobilization. J. Phys. Chem. C. 114, 8353-8362; **(14)** Hung, T.-C., Fu, C.-C., Su, C.-H., Chen, J.-Y., Wu, W.-T., Lin, Y.-S., 2011. Immobilization of cellulase onto electrospun polyacrylonitrile (PAN) nanofibrous membranes and its application to the reducing sugar production from microalgae. Eznyme Microb. Tech. 49, 30-37; **(15)** Jain, P., Wilkins, E.S., 1987. Cellulase immobilized on modified nylon for saccharification of cellulose. Biotechnol. Bioeng. 30, 1057-1062; **(16)** Jones, P. O., Vasudevan, P. T., 2010. Cellulose hydrolysis by immobilized Trichoderma reesei cellulase. Biotechnol. Lett. 32, 103-106; **(17)** Kannan, K. R., Jasra, V., 2011. Improved catalytic hydrolysis of carboxy methyl cellulose using cellulase immobilized on functionalized meso cellular foam. J. Porous Mat. 18, 409-416; **(18)** Li, C., Yoshimoto, M., Fukunaga, K., Nakao, K., 2007. Characterization and immobilization of liposome-bound cellulase for hydrolysis of insoluble cellulose. Bioresour. Technol. 98, 1366-1372; **(19)** Nakamura, Y., Sawada, T., Inoue, E., 2001. Enhanced ethanol production from enzymatically treated steam-exploded rice straw using extractive fermentation. J. Chem. Technol. Biotechnol. 76, 879-884; **(20)** Qi, B.-K., Chen, X.-R., Shen, F., Su, Y., Wan, Y.-H., 2009. Optimization of enzymatic hydrolysis of wheat straw pretreated by alkaline peroxide using response surface methodology. Ind. Eng. Chem. Res. 48, 7346-7353; **(21)** Shafizadeh, F., Bradbury, A. G. W., 1979. Thermal degradation of cellulose in air and nitrogen at low temperatures. J. Appl. Polym. Sci. 23, 1431-1442; **(22)** Shimokawa, T., Ishida, M., Yoshida, S., Nojiri, M., 2009. Effects of growth stage on enzymatic saccharification and simultaneous saccharification and fermentation of bamboo shoots for bioethanol production. Bioresour. Technol. 100, 6651-6654; **(23)** Tébéka, I. R. M., Silva, A. G. L., Petri, D. F. S., 2009. Hydrolytic activity of free and immobilized cellulose. Langmuir 25, 1582-1587 ; **(24)** Vaillant, F., Millan, A., Millan, P., Dornier, M., Decloux, M., Reynes, M., 2000. Co-immobilized pectinlyase and endocellulase on chitin and Nylon supports. Process Biochem. 35, 989-996; **(25)** Wu, C.-C., Cheng, C., 2005. A study of the hydrolysis of waste paper cellulose with a vertically hanging immobilized cellulase reactor and the reuse of the immobilized cellulose. J. Chin. Chem. Soc. 52, 85-95; **(26)** Wu, L., Yuan, X., Shen, J., 2005. Immobilization of cellulase in nanofibrous PVA membranes by electrospinning. J. Membr. Sci. 250, 167-173.

## SUMMARY OF THE INVENTION

An object of the present invention is for immobilized enzymes, their fabrication methods, and reaction systems using the immobilized enzymes, in which the immobilized enzymes can be recovered and reused.

An embodiment of this invention provides a method for producing an immobilized enzyme, comprising the steps of providing a silica, silanizing the silica by a (mercapto)trimethoxysilane ($(CH_3O)_3Si-(CH_2)_n-SH$, where n is an integer), modifying the silanized silica by nano-gold particles, modifying the nano-gold particles modified silica by an amino acid with an amino group, a carboxyl group, and a thiol group, modifying the amino acid modified silica by a peptide-bond coupling agent with a diimide; and chemically bonding an enzyme to the peptide-bond coupling agent modified silica.

Another embodiment of this invention provides an immobilized enzyme having the following structure:

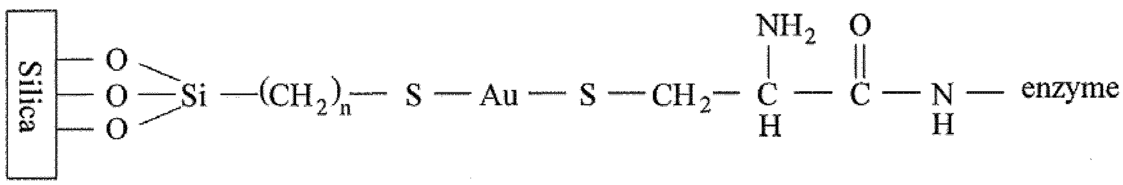

wherein n is an integer.

Another embodiment of this invention provides a reaction system, comprising a bioreactor containing a solution comprising an immobilized enzyme, a substrate tank containing a substrate solution, a first pump for transferring the substrate solution to the bioreactor, such that a substrate of the substrate solution reacts with the immobilized enzyme to proceed a reaction, a second pump transferring the solution out of the bioreactor during the reaction is proceeding, wherein the immobilized enzyme has a structure as described above.

## BRIEF DESCRIPTION OF THE DRAWINGS

Figure 1 shows an immobilized enzyme produced by a method according to a preferred embodiment of the present invention.

Figure 2 shows a reaction system according to an embodiment of the present invention.

Figure 3A-3F show the reusability of the immobilized enzyme of the embodiment of the present invention for three

different batches of immobilized cellulase I, II, and III.

Figure 4A shows the yield of glucose of the three different batches of immobilized cellulase I, II, and III for 3 or 5 cycles of continuous hydrolysis.

Figure 4B shows the overall activity of the three different batches of immobilized cellulase I, II, and III for 3 or 5 cycles of continuous hydrolysis.

## DESCRIPTION OF THE PREFERRED EMBODIMENT

Fig. 1 shows an immobilized enzyme produced by a method according to an embodiment of this invention. In this preferred embodiment, an enzyme, cellulase, is chemically bonded to silica with the assistance of nano-gold particles and other chemical reagents.

As shown in Fig. 1, the first step was the silanization of silica. The silica (10.0 g) was first activated with 200 mL 6 M HCl solution by refluxing at 120°C for 1 day. Then, the activated silica was filtered and cleaned with deionized distilled water until the filtrate is neutral. Then, they were dried at 110°C for 12 h. One gram of the dried and activated silica was silanized with 10.0 mmol (3-mercaptopropyl) trimethoxysilane (MPS) in 150 mL toluene by refluxing at room temperature for 1 day. The silanized silica was filtered, washed with hot toluene, and dried at 110°C for 5 h[12]. In other embodiments, (mercapto)trimethoxysilane ($(CH_3O)_3Si-(CH_2)_n-SH$, wherein n denotes an integer) may replace MPS.

The second step is to modify the silanized silica by nano-gold particles. An apparatus used for nano-gold particles (NAuP) preparation was cleaned with aqua regia ($HCl:HNO_3$ = 3:1 (v/v)) and then with deionized distilled water. One hundred milliliters of 0.2% $HAuCl_4 \cdot 3H_2O$ were refluxed to boil. Then, 2 mL 38.8 mM sodium citrate were added to the boiling $HAuCl_4$ solution to form the nano-gold particles (NAuP). The NAuP solution was cooled to room temperature and the size of NAuP was measured by a particle size analyzer (Brookhaven 90 Plus, New York, USA). Then, the silanized silica was reacted with NAuP at room temperature and shaken for 1 h. The NAuP modified silica was filtered and dried.

In the third step, the NAuP modified silica was chemically bonded with L-cysteine at room temperature and shaken for 1 h. The L-cysteine and NAuP modified silica was filtered, washed with deioniezed distilled water to remove the absorbed L-cysteine, and dried.

In the fourth step, the L-cysteine and NAuP modified silica was further modified by *N,N'*-dicyclohexylcarbodiimide (DCC) in chloroform. The L-cysteine and NAuP modified silica and DCC were reacted for 1 h. The DCC, L-cysteine, and NAuP modified silica was filtered, washed with methanol and deionized distilled water to remove the absorbed DCC, and dried.

In the fifth step, the DCC, L-cysteine, and NAuP modified silica was chemically reacted with cellulase by adding the DCC, L-cysteine, and NAuP modified silica to the cellulase solution for 24 h. The cellulase immobilized silica was then filtered, washed with deionized distilled water, and dried.

It is noted that in this embodiment cellulase is chemically bonded to silica by the assistance of nano-gold particles, L-cysteine, DCC, and so forth. Other embodiments of this invention may use the same bonding mechanism to bond other enzymes to the silica or other material/substrates. In addition, the silica has particle configuration in this embodiment, and it may have other configurations depending on the needs. Further, other amino acid with an amino group, a carboxyl group, and a thiol group may be used to replace L-cysteine, other peptide-bond coupling agent with diimide structure may be used to replace the DCC, and other (mercapto)trimethoxysilane ($(CH_3O)_3Si-(CH_2)_nSH$, wherein n denotes an integer) may be used to replace the MPS.

In this preferred embodiment, the produced immobilized enzyme has the following structure:

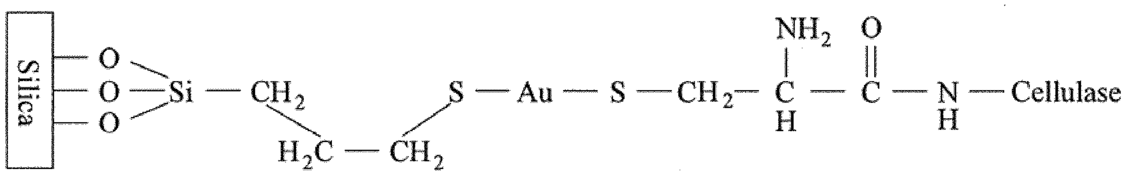

The immobilized enzyme produced by the above embodiment is used to hydrolyze waste bamboo chopsticks powder for investigating its performance. The experiments for hydrolysis are divided into two modes, batch mode and continuous mode.

*Pretreatment of waste bamboo chopsticks*

The waste bamboo chopsticks were immersed in deionized distilled water for 8 h. Then, the chopsticks were taken out from the water and further washed with deionized distilled water. The cleaned chopsticks were dried in oven at 100°C for 8 h. The dried chopsticks were ground into powder by a pulverizer (Rong Tsong, RT-20B, Taichung, Taiwan). The chopsticks powder was stored in the oven at 50°C.

*Batch hydrolysis*

Waste bamboo chopsticks powder (2.5 g) in 1 L deionized distilled water was put in a 3-L bench scale bioreactor and sterilized at 121°C for 30 min. The bamboo chopsticks powder solution was then cooled down to 45°C. An amount of approximately 1.0 g cellulase immobilized silica containing theoretically 40 mg cellulase was weighed and irradiated with UV for 30 min and put in 1 L waste bamboo chopsticks powder solution to perform the hydrolysis. The conditions for the reaction were pH 4.0, 45°C, and an agitation rate of 150 rpm4. During the reaction, 1 mL hydrolysate was sampled at certain time interval for analysis and monitoring the concentration of the carbohydrate products. After the hydrolysis, the cellulase immobilized silica were recovered by filtration, washed with deionized distilled water, and dried at 40°C for 12 h. The recovered immobilized cellulase was stored in the refrigerator at 0°C to restore the activity of the immobilized cellulase. Then, another batch of the waste bamboo chopsticks powder was hydrolyzed by the recovered immobilized cellulase.

*Optimal hydrolysis conditions*

The search of optimal pH for the immobilized cellulase hydrolysis of waste bamboo chopsticks powder was performed by small scale shaker flask experiments. Nine different pHs (pH 3.0, 4.0, 5.0, 6.0, 7.0, 8.0, 9.0, 10.0, and 11.0) were tested with 100 mL solution containing 0.25 g waste bamboo chopsticks powder and 0.1 g cellulase immobilized silica (40 mg cellulase (g silica)-1). Before the reaction, the immobilized cellulase was irradiated with UV for 30 min and the pH was adjusted by 0.01 M H2SO4 and 0.01 N NaOH. The reaction temperature was kept at 45°C and the shaking rate was maintained at 150 rpm. The reaction was performed 2 days. To determine the optimal pH, the carbohydrates produced and the activity of the immobilized cellulase were assayed by High-Performance Liquid Chromatography (HPLC). The optimal pH found was then used for the search of optimal temperature that was performed by 3-L bench scale bioreactor with 1 L working volume containing 2.5 g waste bamboo chopsticks powder for 5 different temperatures (35°C, 40°C, 45°C, 50°C, and 55°C).

*Reaction system*

Fig. 2 shows the schematic assembly of a reaction system 1 according to an embodiment of this invention. In this example, the reaction system 1 is used for continuous hydrolysis of waste bamboo chopsticks powder. A 3-L automatically controlled bench scale bioreactor 10 with 1 L aqueous 0.3 g $L^{-1}$ waste bamboo chopsticks powder suspension and 1.0 g cellulase immobilized silica (40 mg cellulase (g silica)$^{-1}$) was used to perform the continuous hydrolysis for 4 days under the optimal pH and temperature found previously. The immobilized cellulase was irradiated with UV for 30 min prior to use. During the reaction, fresh waste bamboo chopsticks powder suspension (0.2 g $L^{-1}$) was continuously fed to the bioreactor 10 by a peristaltic pump 11 at the rate of 0.5 mL $min^{-1}$. Synchronously, the hydrolysate in the bioreactor 10 was continuously drawn and collected in a collection flask 13 using another peristaltic pump 12 pumped at the same rate (0.5 mL $min^{-1}$). In the meantime, one milliliter hydrolysate can be on-line sampled from the bioreactor 10 using a specific designed sampling and injection device 14 (e.g. a syringe) and on-line injected to the HPLC system 15 for carbohydrate products analysis. The hydrolysate collected in the collection flask 13 was also sampled off-line and analyzed with HPLC 15.

In this embodiment, the 3-L bioreactor 10 is purchased form Bio Top, BTF-A, Taichung, Taiwan. The bioreactor 10 comprises a controller for controlling the agitation rate, temperature, pH, and so on. In addition, two extra peristaltic pumps 11/12 (Longerpump™ BT50-1J, Hebei, China) were provided with the continuous hydrolysis bioreactor 10 to function the continuous feed and draw of the waste bamboo chopsticks powder solution. A hot plate 16 (Coming PC-420, California, USA) was used for keeping the feed reservoir 17 at the desired reaction temperature. The combined glass pH electrode used for monitoring the pH of the reaction was obtained from Mettler (InPro 30301225, Greifensee, Switzerland). The hydrolysis products was analyzed by high-performance liquid chromatography (HPLC) system 15 which consists of a mobile phase delivery pump (Jasco PU-1580, Tokyo, Japan), an injection valve with a 20 $\mu$L sampling loop (Rheodyne 7125, Cotati, California, USA), a column oven (Shimadzu CTO 6A, Kyoto, Japan), a $H^+$ ion-exchange analytical column (Rezex ROA organic acid, 300 $\times$ 7.8 mm i.d., Phenomenex, Torrance, California, USA) protected with a guard column (Rezex 4 $\times$ 3 mm i.d., Phenomenex, Torrance, California, USA), a refractive index (RI) detector (Shimadzu

RID-6A, Kyoto, Japan), and a personal computer including a chromatographic data processing software (SISC, Taipei, Taiwan). The HPLC analysis system 15 can be coupled to the bioreactor 10 through a specific designed syringe device 14[5] and a 3-port valve 18 to perform the on-line monitoring of carbohydrate products.

It is noted that a person skilled in the art may alter, change, replace, add, or reduce the components of the reaction system, according to the variation of situations and needs. In addition, the dimension, parameters, and specification of components of the reaction system 1 may be rescaled according to the needs. The flow rate and reaction parameters may also be adjusted.

The yield for the continuous hydrolysis is calculated according to Eq. 1,

$$\text{Yield} = \frac{W_{\text{glucose in reactor}} + W_{\text{glucose in flask}}}{W_{\text{initial chopsticks powder}} + W_{\text{added chopsticks powder}}} \times 100\% \qquad (1)$$

where $W_{\text{glucose in reactor}}$ (mg) is the amount of glucose produced from the waste bamboo chopsticks powder in the bioreactor 10, $W_{\text{glucose in flask}}$ (mg) is the amount of glucose in the collection flask 13, $W_{\text{initial chopsticks powder}}$ (mg) is the initial amount of waste bamboo chopsticks powder in the bioreactor 10, and $W_{\text{added chopsticks powder}}$ (mg) is the total amount of added waste bamboo chopsticks powder during the 4-day reaction which was calculated by Eq. 2,

$$W_{\text{added chopsticks powder}} = C_{\text{chopsticks powder}} \times V_{\text{flow rate}} \times t \qquad (2)$$

where $C_{\text{chopsticks powder}}$ (mg $L^{-1}$) is the concentration of fresh waste bamboo chopsticks powder in the continuous feed, $V_{\text{flow rate}}$ (mL $min^{-1}$) is the delivery rate of the peristaltic pump 11 for continuously feeding the fresh waste bamboo chopsticks powder suspension to the bioreactor 10, and *t* (min) is the sampling time during the hydrolysis.

After hydrolysis, the immobilized cellulase was recovered by filtration, washed with deionized distilled water, and dried at 40°C for 12 h. Then, the recovered immobilized cellulase was weighed and stored in the refrigerator at 0°C to restore the activity. Another hydrolysis of the waste bamboo chopsticks powder was then performed again using the recovered immobilized cellulase. The overall activity ((mg glucose) $h^{-1}$ (g cellulase)$^{-1}$) of the immobilized cellulase is calculated by Eq. 3,

$$\text{Activity} = W_{\text{total glucose produced}} : t \times W_{\text{cellulase}} \qquad (3)$$

where $W_{\text{glucose in reactor}}$ (mg) is the total amount of glucose produced from waste bamboo chopsticks powder in the bioreactor 10 during the 4-day reaction, *t* (h) is the total hydrolysis time, and $W_{\text{cellulase}}$ (g) is the amount of cellulase used for performing the hydrolysis. The amount of cellulase used for performing the hydrolysis is calculated by Eq. 4,

$$W_{\text{cellulase}} = 0.04 \times \frac{W_{\text{recovered immobilize d cellulase}}}{W_{\text{initial immobilize d cellulase}}} \qquad (4)$$

where 0.04 (g/g) is the theoretical amount of cellulase immobilized on silica, $W_{\text{recovered immobilized cellulase}}$ (g) is the amount of recovered immobilized cellulase, $W_{\text{initial immobilized cellulase}}$ (g) is the amount of initial immobilized cellulase.

*Analysis by HPLC*

One milliliter of the blank solution, i.e. before the addition of immobilized cellulase, and the hydrolysate immediately after the addition of the immobilized cellulase (~5 min) was sampled, then, the sampling being performed every 4 h during the first 8 h of the reaction, every 8 h during the 8th hour to the 48th hour of the reaction, and every 12 h for the last 2 days of the reaction using a specific designed syringe and on-line analyzed with HPLC 15. Simultaneously,

one milliliter of the immobilized cellulase hydrolysate collected in the collection flask 13 was also sampled at the same time using a pipet and analyzed off-line with HPLC 15. The quantification of the carbohydrate product content in the immobilized cellulase hydrolysate was determined by the on-line external calibration method[5]. Because the mobile phase of the HPLC analysis used for carbohydrates produced was pure water, there is no secondary pollution problem from the analysis and is also an advantage for carbohydrate products recovery.

*Result*

In the above-mentioned embodiment, cellulase was successfully chemically bonded on silica through the assistance of nano-gold particle. The average size of nano-gold particles synthesized by the reduction method [24] was 7.3 nm which means a large surface area can be utilized for immobilization. Except trimethoxysilane, all of the materials (silica, gold particle, L-cysteine, and cellulase) used for the immobilization are biocompatible. The preparation procedure is not difficult and most of them can be operated under atmospheric pressure and at room temperature.

The activity of free cellulase and immobilized cellulase for the hydrolysis of waste bamboo chopsticks powder was shown in Table 1. Using the same reaction conditions (pH 4.0 and 45 °C)[5, 8, 25], the activity of free cellulase for glucose production was obviously larger than those of the immobilized cellulase. This phenomenon probably indicates that the mass transfer and the substrate contacting or docking is better for free cellulase hydrolysis than for immobilized cellulase hydrolysis. However, the other possibility for the worse production of glucose with immobilized cellulase is because the reaction conditions (pH 4.0 and 45°C) used for the immobilized cellulase hydrolysis are not optimal. Nevertheless, the results demonstrate that the immobilized cellulase can be reused several times although at a little bit reduced activity. On the other hand, the activity of the reused immobilized cellulase seems being maintained at certain level and does not successively decrease for the repeated hydrolysis which indicates a breakthrough in enzyme reusing.

Table 1[a]

| Cellulase type | Number of reuse | Glucose[b] (ppm) Avg. S.D. | R.S.D. (%) | Xylose[b] (ppm) Avg. S.D. | R.S.D. (%) | Activity of cellulase ((mg glucose) $h^{-1}$ (g cellulase)$^{-1}$) | Yield[c] (%) |
|---|---|---|---|---|---|---|---|
| Free Cellulase | - | 165.9 ± 3.0 | 1.8 | 45.7 ± 3.4 | 7.4 | 86.4 ± 1.6 | 6.6 |
| | 1 | 139.5 ± 2.0 | 1.4 | 17.2 ± 2.3 | 13.4 | 72.7 ± 1.0 | 5.6 |
| Immobilized | 2 | 71.4 ± 2.7 | 3.8 | 4.7 ± 3.3 | 70.2 | 37.2 ± 1.4 | 2.9 |
| Cellulase | 3 | 88.5 ± 2.4 | 2.7 | 3.8 ± 2.5 | 65.8 | 46.1 ± 1.3 | 3.5 |
| | 4 | 92.1 ± 2.4 | 2.6 | 2.1 ± 2.6 | 123.8 | 48.0 ± 1.3 | 3.7 |

[a]Each hydrolysis was performed with 2.5 g waste bamboo chopsticks powder under pH 4.0 and 45°C for 2 days with an enzyme loading of 40 mg free cellulase or 40 mg cellulase (g silica)$^{-1}$.
[b]The analysis was performed by HPLC and each sample was analyzed 4 times (n = 4).
[c]The yield calculated was based on the amount of glucose produced only.

*Optimum conditions for immobilized cellulase hydrolysis*

Since the three dimensional conformation of cellulase can be altered by the immobilization of cellulase on silica, the activity of the immobilized cellulase, thus the optimal reaction conditions for the hydrolysis of waste bamboo chopsticks powder can be changed by the immobilization. Therefore, new optimal pH and temperature for the immobilized cellulase hydrolysis of waste bamboo chopsticks powder should be investigated in order to apply properly for the continuous hydrolysis.

*Optimal pH*

The search of optimal pH for the immobilized cellulase hydrolysis of the waste bamboo chopsticks powder was performed by shaker-flask experiments that keep the hydrolysis temperature at 45°C and varying the pH from pH 3.0

to pH 11.0. The amount of glucose produced and the activity of immobilized cellulase calculated after reaction are shown in Table 2. The amount of glucose produced, thus the yield and the activity of the immobilized cellulase, of the hydrolysis are approximately the same for the reaction pH at 4.0, 7.0, 8.0, and 10.0. However, the amount of glucose produced for the reaction at pH 8.0 is a bit larger than those of the other reaction runs. Therefore, pH 8.0 was selected as the optimal pH and used for the continuous hydrolysis of waste bamboo chopsticks powder by the immobilized cellulase.

Table 2[a]

| pH | Temp. (°C) | Glucose[b] (ppm) Avg. S.D. | R.S.D. (%) | Xylose[b] (ppm) Avg. S.D. | R.S.D. (%) | Activity of cellulase ((mg glucose) $h^{-1}$ (g cellulase)$^{-1}$) | Yield (%) |
|---|---|---|---|---|---|---|---|
| 3.0 | 45.0 | 121.6 ± 2.7 | 2.2 | 11.8 ± 2.6 | 22.0 | 63.3 ± 1.4 | 4.9 |
| 4.0 | 45.0 | 143.1 ± 2.9 | 2.0 | 22.3 ± 3.0 | 13.5 | 74.5 ± 1.5 | 5.7 |
| 5.0 | 45.0 | 134.8 ± 2.8 | 2.1 | 11.6 ± 3.0 | 25.9 | 70.2 ± 1.5 | 5.4 |
| 6.0 | 45.0 | 137.5 ± 2.8 | 2.0 | 12.2 ± 3.0 | 24.6 | 71.6 ± 1.5 | 5.5 |
| 7.0 | 45.0 | 143.1 ± 2.8 | 2.0 | 14.3 ± 3.2 | 22.4 | 74.5 ± 1.5 | 5.7 |
| 8.0 | 45.0 | 146.1 ± 2.8 | 1.9 | 14.4 ± 2.9 | 20.1 | 76.1 ± 1.5 | 5.8 |
| 9.0 | 45.0 | 135.4 ± 2.8 | 2.1 | 8.4 ± 2.8 | 33.3 | 70.5 ± 1.5 | 5.4 |
| 10.0 | 45.0 | 143.4 ± 2.8 | 2.0 | 10.9 ± 3.0 | 27.5 | 74.7 ± 1.5 | 5.7 |
| 11.0 | 45.0 | 133.6 ± 2.8 | 2.1 | 7.9 ± 3.7 | 46.8 | 69.6 ± 1.5 | 5.3 |

[a]Each hydrolysis was performed with 2.5 g waste bamboo chopsticks powder for 2 days with an enzyme loading of 40 mg cellulase (g silica)$^{-1}$.
[b]The analysis was performed by HPLC and each sample was analyzed 4 times (*n* = 4).
[c]The yield calculated was based on the amount of glucose produced only.

*Optimal temperature*

The search of optimal temperature was performed by a 3-L bench scale batch type hydrolysis at pH 8.0. Five different temperatures (35°C, 40°C, 45°C, 50°C, and 55°C) were tested individually for the batch type immobilized cellulase hydrolysis of waste bamboo chopsticks powder. Table 3 shows the amount of glucose produced and the calculated cellulase activity. Obviously, the amount of glucose produced, the yield, and the cellulase activity were greatest for the reaction at temperature 50°C. Therefore, the optimal temperature selected for the study of continuous hydrolysis is 50°C.

Table 3[a]

| Temp. (°C) | pH | Glucose[b] (ppm) Avg. S.D. | R.S.D. (%) | Xylose[b] (ppm) Avg. S.D. | R.S.D. (%) | Activity of cellulase ((mg glucose) $h^{-1}$ (g cellulase)$^{-1}$) | Yield[c] (%) |
|---|---|---|---|---|---|---|---|
| 35 | 8.0 | 137.1 ± 2.4 | 1.8 | 9.9 ± 2.5 | 25.3 | 71.4 ± 1.3 | 5.5 |
| 40 | 8.0 | 151.5 ± 2.5 | 1.7 | 9.7 ± 2.5 | 25.8 | 78.9 ± 1.3 | 6.1 |
| 45 | 8.0 | 166.7 ± 2.4 | 1.4 | 15.6 ± 2.5 | 16.0 | 86.8 ± 1.3 | 6.7 |
| 50 | 8.0 | 186.1 ± 2.4 | 1.3 | 21.3 ± 2.5 | 11.7 | 96.9 ± 1.3 | 7.4 |
| 55 | 8.0 | 175.9 ± 2.4 | 1.4 | 16.6 ± 2.5 | 15.1 | 91.6 ± 1.3 | 7.0 |

[a]Each hydrolysis was performed with 2.5 g waste bamboo chopsticks powder for 2 days with an enzyme loading of 40 mg cellulase (g silica)$^{-1}$.
[b]The analysis was performed by HPLC and each sample was analyzed 4 times (n = 4).
[c]The yield calculated was based on the amount of glucose produced only.

*Initial amount of waste bamboo chopsticks powder*

Table 4 shows that the total amount of glucose produced for the batch hydrolysis with an initial 2.5 g $L^{-1}$ waste

bamboo chopsticks powder was 186.6 mg which corresponds to a very low yield of 7.5%. However, as we reduced the initial concentration of waste bamboo chopsticks powder to 0.3 g $L^{-1}$ and repeated the batch hydrolysis, the total amount of glucose produced was 137.8 mg with a corresponding yield of 45.9%. These results indicates that the initial concentration of waste bamboo chopsticks powder cannot be too large for the present enzyme loading of 40 mg cellulose (g silica)$^{-1}$. Therefore, for the continuous hydrolysis of waste bamboo chopsticks powder using the same enzyme loading, the suitable initial concentration of waste bamboo chopsticks powder was searched by a series of experiments by varying the initial substrate concentration and the continuous feed concentration but keeping the continuous feed rate and draw rate at constant. In Table 4, results of the continuous hydrolysis obviously indicate that the glucose production yield increases with a decreasing of the initial substrate concentration. However, results in Table 4 shows that the continuous hydrolysis with an initial substrate concentration of 0.5 g $L^{-1}$ and a continuous substrate feed concentration of 0.2 g $L^{-1}$ has the largest total amount (683.1 mg) of glucose produced but the yield is only 63.5% which means a large amount of the waste bamboo chopsticks powder was not in use thus wasted. On the contrary, although the continuous hydrolysis with an initial substrate concentration of 0.2 g $L^{-1}$ and a continuous substrate feed concentration of 0.2 g $L^{-1}$ has a greatest yield (82.6%) but the total amount of glucose produced (641.1 mg) is not maximal which means the activity of the immobilized cellulase is small. Therefore, in order to have a lower wasting rate of the substrate and a greater immobilized cellulase activity or yield for glucose production, the continuous hydrolysis with the initial substrate concentration of 0.3 g $L^{-1}$ is chosen for our later experiments that has a reasonable large immobilized cellulase activity to gives large amount of glucose (649.1 mg) and yield (74.1 %). Also, the two runs with high initial substrate concentration (1.0 and 2.5 g $L^{-1}$) give a low yield which indicates that substrate inhibition should exist in this type of immobilized cellulase hydrolysis.

Table 4[a]

| Initial concentration of bamboo chopsticks powder (g $L^{-1}$) | Feed concentration of bamboo chopsticks powder (g $L^{-1}$) | Feed and draw rate (mL $min^{-1}$) | Glucose in bioreactor[b] (mg) | | Glucose in collection flask[b] (mg) | | Activity of cellulase ((mg glucose) $h^{-1}$ (g cellulase)$^{-1}$) | Yield[c] (%) |
|---|---|---|---|---|---|---|---|---|
| | | | Avg. S.D. | R.S.D. (%) | Avg. S.D. | R.S.D. (%) | | |
| 2.5 | - | - | 186.6 ± 2.5 | 1.3 | - | - | 48.6 | 7.5 |
| 0.3 | - | - | 137.8 ± 2.5 | 1.8 | - | - | 35.9 | 45.9 |
| 0.2 | 0.2 | 0.5 | 166.4 ± 2.4 | 1.4 | 474.7 ± 6.9 | 1.5 | 43.3 | 82.6 |
| 0.3 | 0.2 | 0.5 | 167.8 ± 2.4 | 1.4 | 481.3 ± 6.8 | 1.4 | 43.7 | 74.1 |
| 0.5 | 0.2 | 0.5 | 173.7 ± 2.5 | 1.4 | 509.4 ± 6.9 | 1.4 | 45.2 | 63.5 |
| 1.0 | 0.2 | 0.5 | 168.4 ± 2.4 | 1.4 | 448.5 ± 6.9 | 1.5 | 43.9 | 39.2 |
| 2.5 | 0.5 | 0.5 | 161.9 ± 2.4 | 1.5 | 412.1 ± 6.8 | 1.7 | 42.2 | 23.0 |

[a]Each hydrolysis was performed at pH 8.0 and 50°C for 4 days with an enzyme loading of 40 mg cellulase (g silica)$^{-1}$.
[b]The analysis was performed by HPLC and each sample was analyzed 4 times (n = 4).
[c]The Yield is defined in the previously mentioned equation 1.

*Reusability of immobilized cellulase*

The reusability of immobilized cellulase for the continuous hydrolysis of waste bamboo chopsticks powder is confirmed by the experimental results shown in Fig. 3A-3F. Three different batches of immobilized cellulase, designated as immobilized cellulase I, II, and III, were prepared and repeatedly used for the continuous hydrolysis with the optimal reaction conditions and operation parameters described before. The results of glucose production in the bioreactor for the continuous hydrolysis of waste bamboo chopsticks powder by different batches of immobilized cellulase are shown in Fig. 3A, 3B, and 3C. The continuous hydrolysis performed by immobilized cellulase I, II, and III was separately repeatedly used 5, 3, and 3 cycles, respectively. Generally, a steady state and a maximum glucose concentration can be reached during the $8^{th}$ hour to $24^{th}$ hour reaction time. The maximum concentration of glucose produced in the bioreactor was in the range 167.8-182.7 mg $L^{-1}$ for a 4-day reaction period. The total amount of glucose produced, i.e. the combination of the amount of glucose in the bioreactor and in the collection flask, for the continuous hydrolysis of waste bamboo chopsticks powder by different batches of immobilized cellulase are illustrated in Fig. 3D, 3E, and 3F. When the hydrolysis reaches a steady state, the accumulation rate of the total amount of glucose was constant. The total amount of glucose accumulated in a 4-day reaction period for the three batches of repeatedly used immobilized cellulase was in the range 637.5-671.2 mg which correspond a yield of 72.8-76.6%. However, a few amount of glucose (6.6-8.6 mg) were generated from the blank hydrolysis (hydrolysis without immobilized cellulase) of waste bamboo chopsticks powder at the reaction conditions pH 8.0 and 50°C. Since the glucose production rate of the immobilized cellulase hydrolysis is about 15 to 20 times greater than that of the blank hydrolysis, the amount of glucose released is mainly due to the cellulase catalyzed hydrolysis.

Fig. 4A shows the variation of yields for the three batches of immobilized cellulase and their repeated hydrolyses. The immobilized cellulase I (the first batch) has been repeatedly used 5 cycles; however, the yield for the recovered and reused cellulase did not decrease significantly but even increases after the second use. The same situation happened for immobilized cellulase II (the second batch) and III (the third batch) although both of them were just repeatedly used 3 cycles. The calculated activities corresponding to each immobilized cellulase used for the hydrolysis are shown in Fig. 4B which clearly explains the variation of the yields. The increase of activities of the immobilized cellulase perhaps is caused by the residue waste bamboo chopsticks powder left with the recovered immobilized cellulase which induces a larger initial activity than previous run thus makes a reduced effect of substrate inhibition. The results shown here thereby indicate that the immobilized cellulase can be used forever with no loss of activity or even better activity and the proposed method cellulase immobilization is reproducible. These two superiorities as far as we know are the first report in literature[1-2, 10-13, 16, 18, 22-26]. Thus, the proposed immobilization method makes the glucose production from waste renewable lignocellulosic biomass competitive in industrial applications.

The embodiment of this invention discloses that an enzyme, such as cellulase, is immobilized to silica with the assistance of nano-gold particles and other reagents. The reusability of the immobilized cellulase was demonstrated by the repeated continuous hydrolysis of waste bamboo chopsticks powder which indicates that the immobilized cellulase can be easily recovered by filtration and reused at least 5 cycles with a recovered activity approximately the same or even better than previous run at pH 8.0 and 50°C. The glucose production for the continuous hydrolysis of waste bamboo chopsticks is greatly improved as compared with batch hydrolysis. Therefore, the glucose production cost from lignocellulosic materials is greatly reduced that makes the biocatalytic process competitive in industrial applications.

## Claims

**1.** A method for producing an immobilized enzyme, comprising the steps of:

providing a silica;
silanizing the silica by a (mercapto)trimethoxysilane ($(CH_3O)_3Si\text{-}(CH_2)_n\text{-}SH$, where n is an integer);
modifying the silanized silica by reacting the silanized silica with nano-gold particles;
modifying the nano-gold particles modified silica by an amino acid with an amino group, a carboxyl group, and a thiol group;
modifying the amino acid modified silica by a peptide-bond coupling agent with a diimide;
chemically bonding an enzyme to the peptide-bond coupling agent modified silica;
wherein the (mercapto)trimethoxysilane ($(CH_3O)_3Si\text{-}(CH_2)_n\text{-}SH$) is (3-mercaptopropyl) trimethoxysilane, the amino acid is L-cysteine, the peptide-bond coupling agent is *N,N'*-dicyclohexylcarbodiimide, and the enzyme is cellulase.

**2.** The method as recited in claim 1, wherein the immobilized enzyme reacts with a substrate, and the reacted immobilized enzyme is recovered for reusing by filtering, cleaning, drying, and refrigerating.

**3.** The method as recited in claim 1, wherein the activity of the recovered immobilized enzyme is not reduced for at least 5 repeated uses by comparing with the activity of the first-time use of the immobilized enzyme, and the activity is defined as: $\text{Activity} = \frac{W_{\text{total glucose produced}}}{t \times W_{\text{cellulase}}}$, wherein $W_{\text{total glucose produced}}$ is a total amount of glucose produced from a cellulose source by a continuous hydrolysis reaction, i.e. a total amount of glucose in a bioreactor plus a total amount of glucose in a collection flask that connects to the bioreactor, *t* is a total hydrolysis time, and $W_{\text{cellulase}}$ is an amount of immobilized cellulase used for the continuous hydrolysis reaction.

**4.** An immobilized enzyme having the following structure:

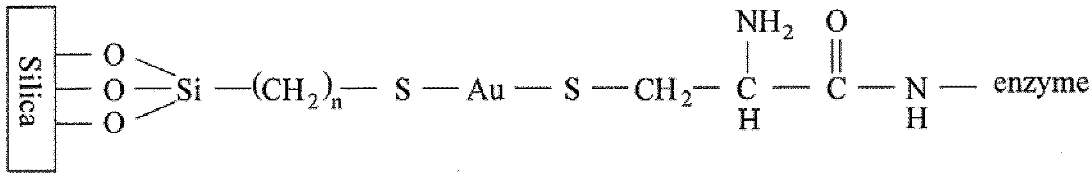

wherein n is an integer.

**5.** The immobilized enzyme as recited in claim 4, wherein n is 3.

**6.** The immobilized enzyme as recited in claim 4, wherein the enzyme is cellulase.

**7.** The immobilized enzyme as recited in claim 4, wherein the immobilized enzyme reacts with a substrate and the reacted immobilized enzyme is recovered for reusing by filtering, cleaning, drying, and refrigerating.

**8.** The immobilized enzyme as recited in claim 4, wherein the enzyme is cellulase and the activity of the recovered immobilized enzyme is not reduced for at least 5 repeated uses by comparing with the activity of the first-time use of the immobilized enzyme, and the activity is defined as: $\text{Activity} = \frac{W_{\text{total glucose produced}}}{t \times W_{\text{cellulase}}}$, wherein $W_{\text{total glucose produced}}$ is a total amount of glucose produced from a cellulose source by a continuous hydrolysis reaction, i.e. a total amount of glucose in a bioreactor plus a total amount of glucose in a collection flask that connects to the bioreactor, *t* is a total hydrolysis time, and $W_{\text{cellulase}}$ is an amount of immobilized cellulase used for the continuous hydrolysis reaction.

**9.** A reaction system, comprising:

a bioreactor containing a solution comprising an immobilized enzyme;
a substrate tank containing a substrate solution;

a first pump for continuously transferring the substrate solution to the bioreactor, such that a substrate of the substrate solution reacts with the immobilized enzyme to proceed a reaction;
a second pump for continuously transferring the solution out of the bioreactor during the reaction;
wherein the immobilized enzyme has the following structure:

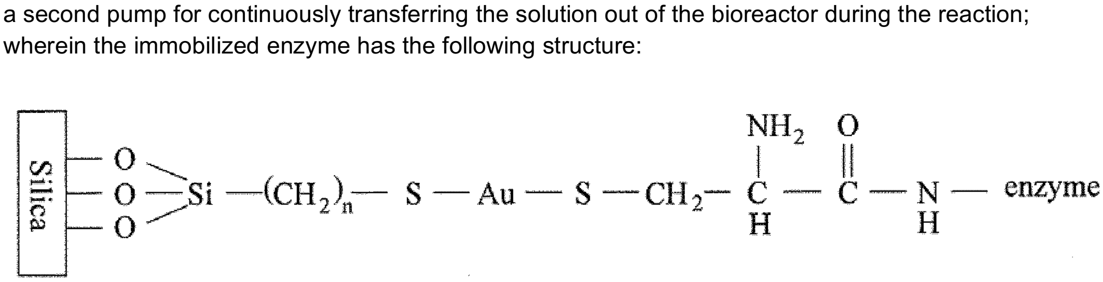

wherein n is an integer.

**10.** The reaction system as recited in claim 9, wherein the enzyme is cellulase.

**11.** The reaction system as recited in claim 9, wherein the enzyme is cellulase, the reacted immobilized enzyme is recovered for reusing by filtering, cleaning, drying, and refrigerating, and the activity of the recovered immobilized enzyme is not reduced for at least 5 repeated uses by comparing with the activity of the first-time use of the immobilized enzyme, and the activity is defined as: $\text{Activity} = \frac{W_{\text{total glucose produced}}}{t \times W_{\text{cellulase}}}$, wherein $W_{\text{total glucose produced}}$ is a total amount of glucose produced from a cellulose source by a continuous hydrolysis reaction, i.e. a total amount of glucose in the bioreactor plus a total amount of glucose in a collection flask that connects to the bioreactor, *t* is a total hydrolysis time, and $W_{\text{cellulase}}$ is an amount of immobilized cellulase used for the continuous hydrolysis reaction.

## Patentansprüche

**1.** Verfahren zum Herstellen eines immobilisierten Enzyms, umfassend die Schritte:

Bereitstellen eines Siliciumdioxids;
Silanisieren des Siliciumdioxids durch ein (Mercapto)trimethoxysilan ($(CH_3O)_3Si\text{-}(CH_2)_n\text{-}SH$, wobei n eine ganze Zahl ist);
Modifizieren des silanisierten Siliciumdioxids durch Zur-Reaktion-Bringen des silanisierten Siliciumdioxids mit Nano-Goldteilchen;
Modifizieren des durch Nano-Goldteilchen modifizierten Siliciumdioxids durch eine Aminosäure mit einer Aminogruppe, einer Carboxylgruppe und einer Thiolgruppe;
Modifizieren des durch Aminosäure modifizierten Siliciumdioxids durch ein Peptidbindung-Kopplungsmittel mit einem Diimid;
chemisches Binden eines Enzyms an das durch Peptidbindung-Kopplungsmittel modifizierte Siliciumdioxid;
wobei das (Mercapto)trimethoxysilan ($(CH_3O)_3Si\text{-}(CH_2)_n\text{-}SH$) (3-Mercaptopropyl)trimethoxysilan ist, die Aminosäure L-Cystein ist, das Peptidbindung-Kopplungsmittel *N,N'*-Dicyclohexylcarbodiimid ist und das Enzym Cellulase ist.

**2.** Verfahren wie vorgetragen in Anspruch 1, wobei das immobilisierte Enzym mit einem Substrat reagiert und das zur Reaktion gebrachte immobilisierte Enzym durch Filtrieren, Reinigen, Trocknen und Kühlen zum Wiederbenutzen rückgewonnen wird.

**3.** Verfahren wie vorgetragen in Anspruch 1, wobei die Aktivität des rückgewonnenen immobilisierten Enzyms beim Vergleichen mit der Aktivität der erstmaligen Benutzung des immobilisierten Enzyms während mindestens 5 wiederholter Benutzungen nicht verringert ist und die Aktivität definiert ist als: $\text{Aktivität} = \frac{W_{\text{gesamte hergestellte Glucose}}}{t \times W_{\text{Cellulase}}}$, wobei $W_{\text{gesamte hergestellte Glucose}}$ eine Gesamtmenge von Glucose ist, die durch eine kontinuierliche Hydrolysereaktion aus einer Cellulosequelle hergestellt wird, d.h. eine Gesamtmenge von Glucose in einem Bioreaktor zuzüglich einer Gesamtmenge von Glucose in einem Auffangkolben, der an den Bioreaktor angeschlossen ist, *t* eine Gesamthydrolysedauer ist und $W_{\text{Gellulase}}$ eine Menge von immobilisierter Cellulase ist, die für die kontinuierliche Hydrolysereaktion benutzt wird.

**4.** Immobilisiertes Enzym mit der folgenden Struktur:

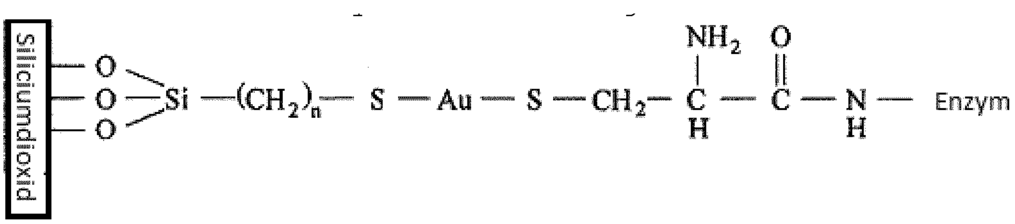

wobei n eine ganze Zahl ist.

**5.** Immobilisiertes Enzym wie in Anspruch 4 vorgetragen, wobei n 3 ist.

**6.** Immobilisiertes Enzym wie in Anspruch 4 vorgetragen, wobei das Enzym Cellulase ist.

**7.** Immobilisiertes Enzym wie in Anspruch 4 vorgetragen, wobei das immobilisierte Enzym mit einem Substrat reagiert und das zur Reaktion gebrachte immobilisierte Enzym durch Filtrieren, Reinigen, Trocknen und Kühlen zum Wiederbenutzen rückgewonnen wird.

**8.** Immobilisiertes Enzym wie in Anspruch 4 vorgetragen, wobei das Enzym Cellulase ist und die Aktivität des rückgewonnenen immobilisierten Enzyms beim Vergleichen mit der Aktivität der erstmaligen Benutzung des immobilisierten Enzyms während mindestens 5 wiederholter Benutzungen nicht verringert ist und die Aktivität definiert ist als: $\text{Aktivität} = \frac{W_{\text{gesamte hergestellte Glucose}}}{t \times W_{\text{Cellulase}}}$, wobei $W_{\text{gesamte hergestellte Glucose}}$ eine Gesamtmenge von Glucose ist, die durch eine kontinuierliche Hydrolysereaktion aus einer Cellulosequelle hergestellt wird, d.h. eine Gesamtmenge von Glucose in einem Bioreaktor zuzüglich einer Gesamtmenge von Glucose in einem Auffangkolben, der an den Bioreaktor angeschlossen ist, t eine Gesamthydrolysedauer ist und $W_{\text{Cellulase}}$ eine Menge von immobilisierter Cellulase ist, die für die kontinuierliche Hydrolysereaktion benutzt wird.

**9.** Reaktionssystem, umfassend:

einen Bioreaktor, der eine Lösung enthält, die ein immobilisiertes Enzym umfasst;
einen Substrattank, der eine Substratlösung enthält;
eine erste Pumpe zum kontinuierlichen Überführen der Substratlösung zu dem Bioreaktor, sodass ein Substrat der Substratlösung mit dem immobilisierten Enzym reagiert, um eine Reaktion ablaufen zu lassen;
eine zweite Pumpe zum kontinuierlichen Überführen der Lösung während der Reaktion aus dem Bioreaktor heraus;
wobei das immobilisierte Enzym die folgende Struktur aufweist:

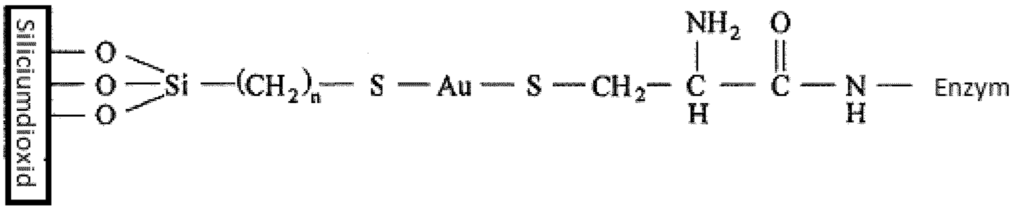

wobei n eine ganze Zahl ist.

**10.** Reaktionssystem wie in Anspruch 9 vorgetragen, wobei das Enzym Cellulase ist.

**11.** Reaktionssystem wie in Anspruch 9 vorgetragen, wobei das Enzym Cellulase ist, das zur Reaktion gebrachte immobilisierte Enzym durch Filtrieren, Reinigen, Trocknen und Kühlen zum Wiederbenutzen rückgewonnen wird und die Aktivität des rückgewonnenen immobilisierten Enzyms beim Vergleichen mit der Aktivität der erstmaligen Benutzung des immobilisierten Enzyms während mindestens 5 wiederholter Benutzungen nicht verringert ist und die Aktivität definiert ist als: $\text{Aktivität} = \frac{W_{\text{gesamte hergestellte Glucose}}}{t \times W_{\text{Cellulase}}}$, wobei $W_{\text{gesamte hergestellte Glucose}}$ eine Gesamtmenge von Glucose ist, die durch eine kontinuierliche Hydrolysereaktion aus einer Cellulosequelle hergestellt wird, d.h. eine Gesamtmenge von Glucose in dem Bioreaktor zuzüglich einer Gesamtmenge von Glucose in einem Auffangkolben, der an den Bioreaktor angeschlossen ist, t eine Gesamthydrolysedauer ist und $W_{\text{Cellulase}}$ eine Menge von immobilisierter Cellulase ist, die für die kontinuierliche Hydrolysereaktion benutzt wird.

## Revendications

**1.** Procédé de production d’une enzyme immobilisée, comprenant les étapes consistant à :

fournir une silice ;
silaniser la silice à l'aide d'un (mercapto) triméthoxysilane ($(CH_3O)_3Si-(CH_2)_n-SH$, où n représente un nombre entier) ;
modifier la silice silanisée en faisant réagir la silice silanisée avec des nanoparticules d'or ;
modifier la silice modifiée contenant des nanoparticules d'or par un acide aminé avec un groupe amino, un groupe carboxylate, et un groupe thiol ;
modifier la silice modifiée contenant un acide aminé par un agent de couplage de liaison peptidique avec un diimide ;
lier de manière chimique une enzyme à la silice modifiée contenant un agent de couplage de liaison peptidique ;
dans lequel le (mercapto) triméthoxysilane ($(CH_3O)_3Si-(CH_2)_n-SH$) est le (3-mercaptopropyl) triméthoxysilane, l'acide aminé est la L-cystéine, l'agent de couplage de liaison peptidique est le *N,N'*-dicyclohexylcarbodiimide, et l'enzyme est la cellulase.

**2.** Procédé selon la revendication 1, dans lequel l'enzyme immobilisée réagit avec un substrat, et l'enzyme immobilisée qui a réagi est récupérée pour être réutilisée par filtration, nettoyage, séchage et réfrigération.

**3.** Procédé selon la revendication 1, dans lequel l'activité de l'enzyme immobilisée qui a été récupérée n'est pas réduite pendant au moins 5 usages répétés par rapport à l'activité de première utilisation de l'enzyme immobilisée, et l'activité est définie comme : $Activit\acute{e} = \frac{W\ glucose\ total\ produit}{t \times W\ cellulase}$, dans laquelle $W_{glucose\ total\ produit}$ représente une quantité totale de glucose produit à partir d'une source de cellulose par une réaction d'hydrolyse continue, c'est-à-dire, une quantité totale de glucose dans un bioréacteur plus une quantité totale de glucose dans un flacon de collecte qui se connecte au bioréacteur, t représente un temps d'hydrolyse total, et $W_{cellulase}$ représente une quantité de cellulase immobilisée utilisée pour la réaction d'hydrolyse continue.

**4.** Enzyme immobilisée possédant la structure suivante :

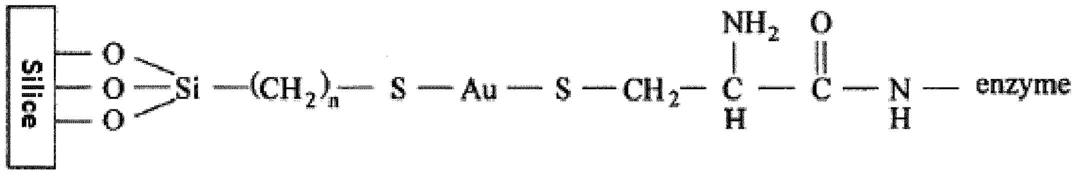

dans laquelle n représente un nombre entier.

**5.** Enzyme immobilisée selon la revendication 4, dans laquelle n vaut 3

**6.** Enzyme immobilisée selon la revendication 4, dans laquelle l'enzyme est la cellulase.

**7.** Enzyme immobilisée selon la revendication 4, dans laquelle l'enzyme immobilisée réagit avec un substrat et l'enzyme immobilisée qui a réagi est récupérée pour être réutilisée par filtration, nettoyage, séchage et réfrigération.

**8.** Enzyme immobilisée selon la revendication 4, dans laquelle l'enzyme est la cellulase et l'activité de l'enzyme immobilisée qui est récupérée n'est pas réduite pendant au moins 5 usages répétés par rapport à l'activité de première utilisation de l'enzyme immobilisée, et l'activité est définie comme : $Activit\acute{e} = \frac{W\ glucose\ total\ produit}{t \times W\ cellulase}$, dans laquelle $W_{glucose\ total\ produit}$ représente une quantité totale de glucose produit à partir d'une source de cellulose par une réaction d'hydrolyse continue, c'est-à-dire, une quantité totale de glucose dans un bioréacteur plus une quantité totale de glucose dans un flacon de collecte qui se connecte au bioréacteur, t représente un temps d'hydrolyse total, et $W_{cellulase}$ représente une quantité de cellulase immobilisée utilisée pour la réaction d'hydrolyse continue.

**9.** Système de réaction, comprenant :

un bioréacteur contenant une solution comprenant une enzyme immobilisée ;
un réservoir de substrat contenant une solution de substrat ;

une première pompe pour transférer de manière continue la solution de substrat au bioréacteur, de sorte qu'un substrat de la solution de substrat réagisse avec l'enzyme immobilisée pour effectuer une réaction ;

une seconde pompe pour transférer de manière continue la solution à l'extérieur du bioréacteur pendant la réaction ;

dans lequel l'enzyme immobilisée possède la structure suivante :

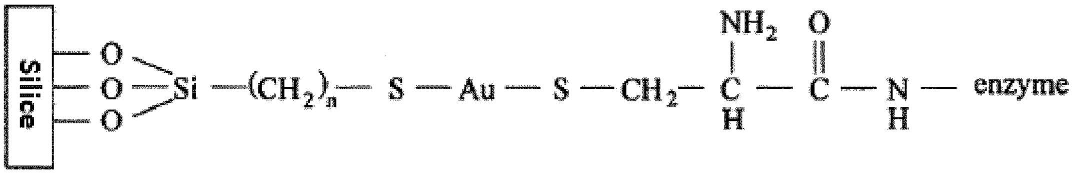

dans laquelle n représente un nombre entier.

**10.** Système de réaction selon la revendication 9, dans lequel l'enzyme est la cellulase.

**11.** Système de réaction selon la revendication 9, dans lequel l'enzyme est la cellulase, l'enzyme immobilisée qui a réagi est récupérée pour être réutilisée par filtration, nettoyage, séchage et réfrigération, et l'activité de l'enzyme immobilisée qui a été récupérée n'est pas réduite pendant au moins 5 usages répétés par rapport à l'activité de première utilisation de l'enzyme immobilisée, et l'activité est définie comme : $Activité = \frac{W\,glucose\,total\,produit}{t \times W\,cellulase}$, dans laquelle $W_{glucose\ total\ produit}$ représente une quantité totale de glucose produit à partir d'une source de cellulose par une réaction d'hydrolyse continue, c'est-à-dire, une quantité totale de glucose dans le bioréacteur plus une quantité totale de glucose dans un flacon de collecte qui se connecte au bioréacteur, *t* représente un temps d'hydrolyse total, et $W_{cellulase}$ représente une quantité de cellulase immobilisée utilisée pour la réaction d'hydrolyse continue.

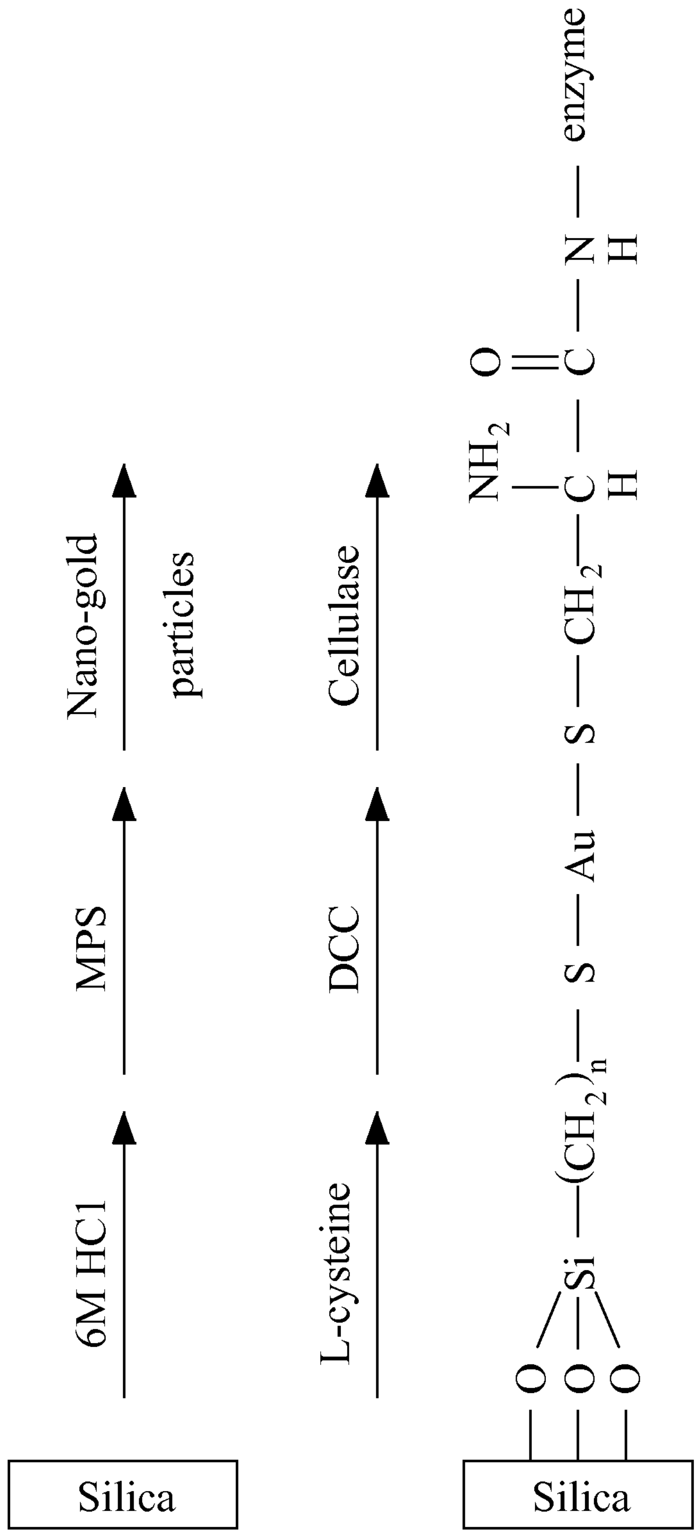


FIG. 1

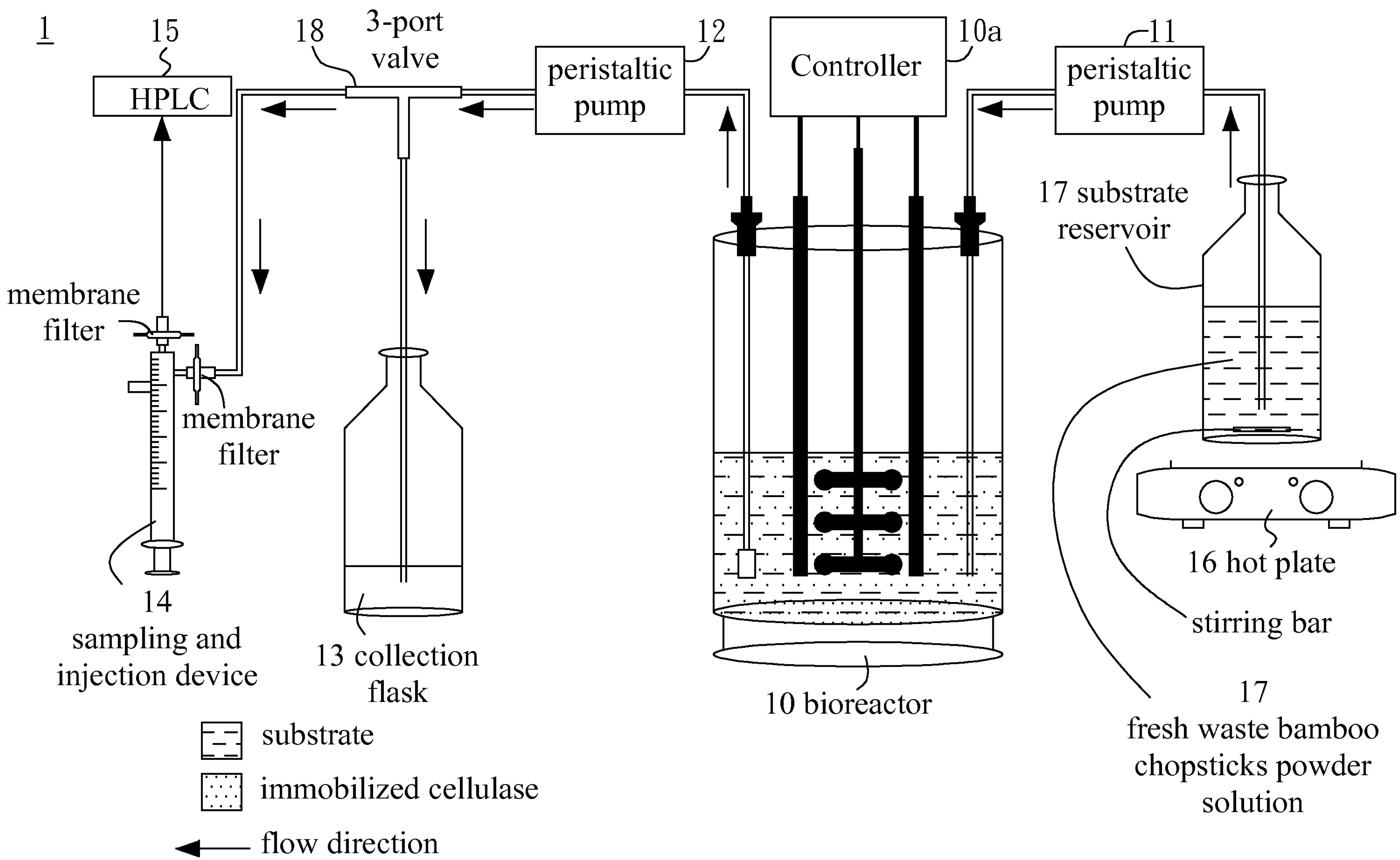
1
15
HPLC
18
3-port valve
12
peristaltic pump
Controller
10a
11
peristaltic pump
17 substrate reservoir
membrane filter
membrane filter
16 hot plate
stirring bar
14
sampling and injection device
13 collection flask
10 bioreactor
17
fresh waste bamboo chopsticks powder solution
substrate
immobilized cellulase
flow direction

FIG. 2

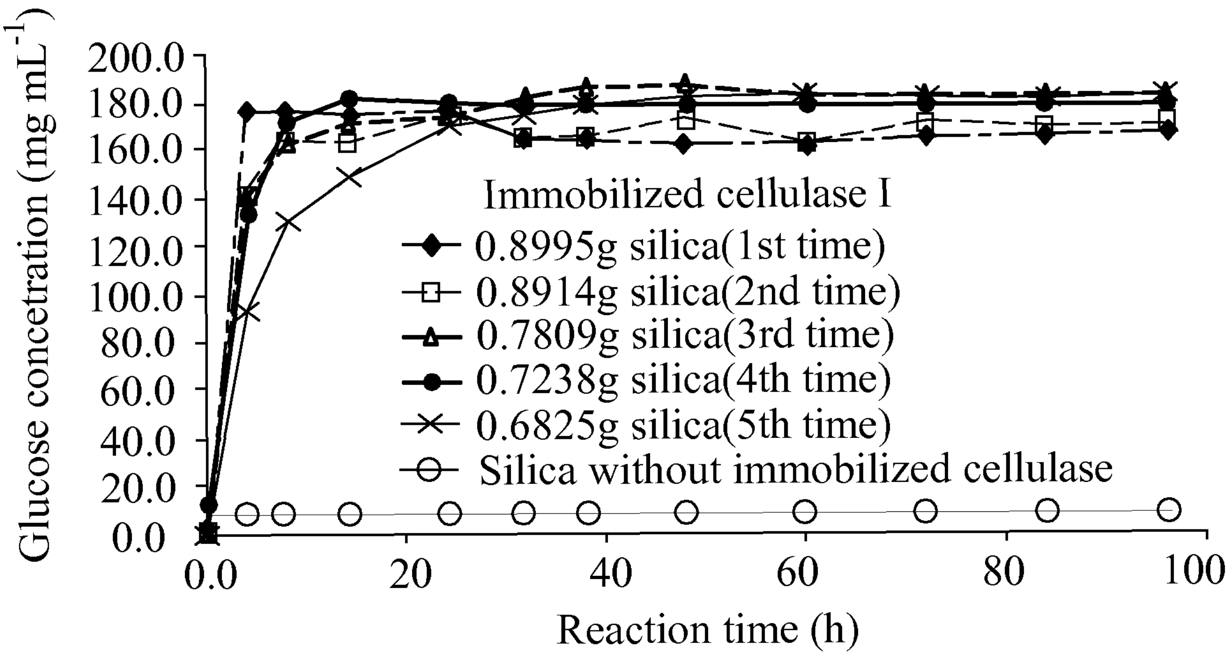
Glucose concetration (mg mL-1)
200.0
180.0
160.0
140.0
120.0
100.0
80.0
60.0
40.0
20.0
0.0
0.0
20
40
60
80
100
Reaction time (h)
Immobilized cellulase I
0.8995g silica(1st time)
0.8914g silica(2nd time)
0.7809g silica(3rd time)
0.7238g silica(4th time)
0.6825g silica(5th time)
Silica without immobilized cellulase

FIG. 3A

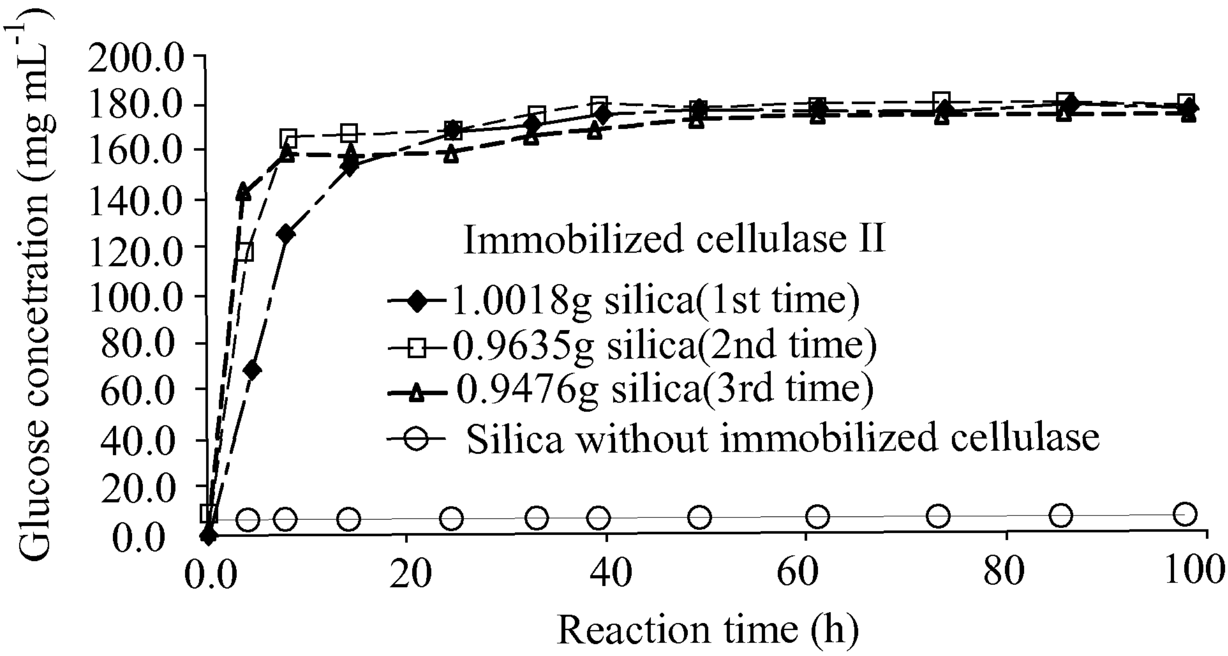
Glucose concetration (mg mL-1)
200.0
180.0
160.0
140.0
120.0
100.0
80.0
60.0
40.0
20.0
0.0
0.0
20
40
60
80
100
Reaction time (h)
Immobilized cellulase II
1.0018g silica(1st time)
0.9635g silica(2nd time)
0.9476g silica(3rd time)
Silica without immobilized cellulase

FIG. 3B

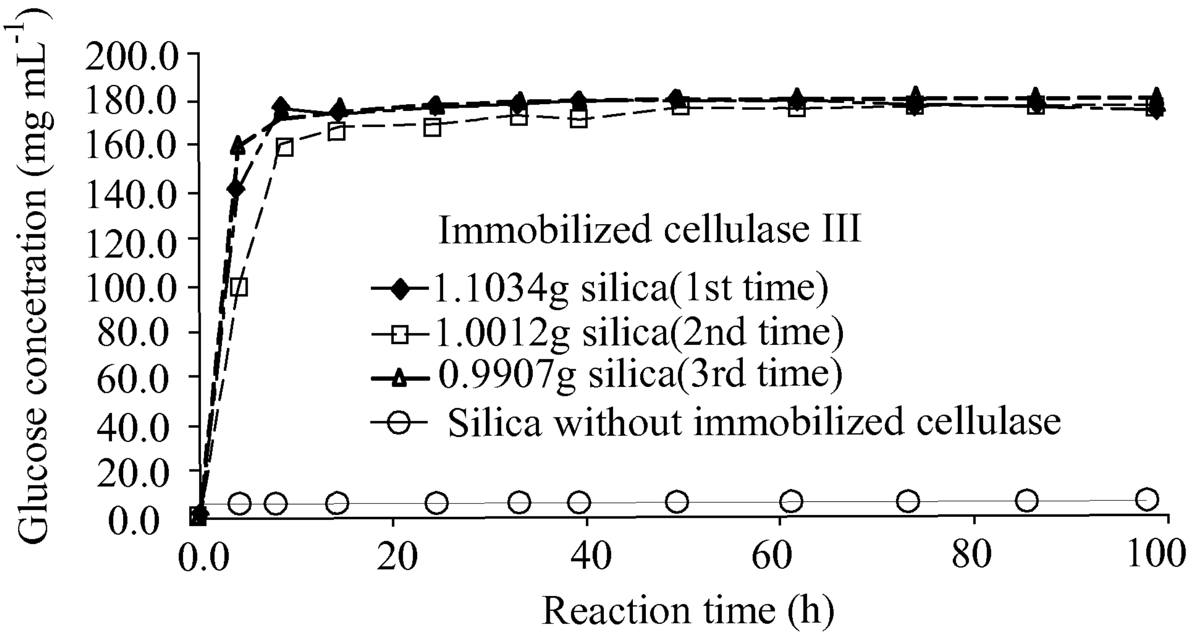
Glucose concetration (mg mL-1)
200.0
180.0
160.0
140.0
120.0
100.0
80.0
60.0
40.0
20.0
0.0
0.0
20
40
60
80
100
Reaction time (h)
Immobilized cellulase III
1.1034g silica(1st time)
1.0012g silica(2nd time)
0.9907g silica(3rd time)
Silica without immobilized cellulase

FIG. 3C

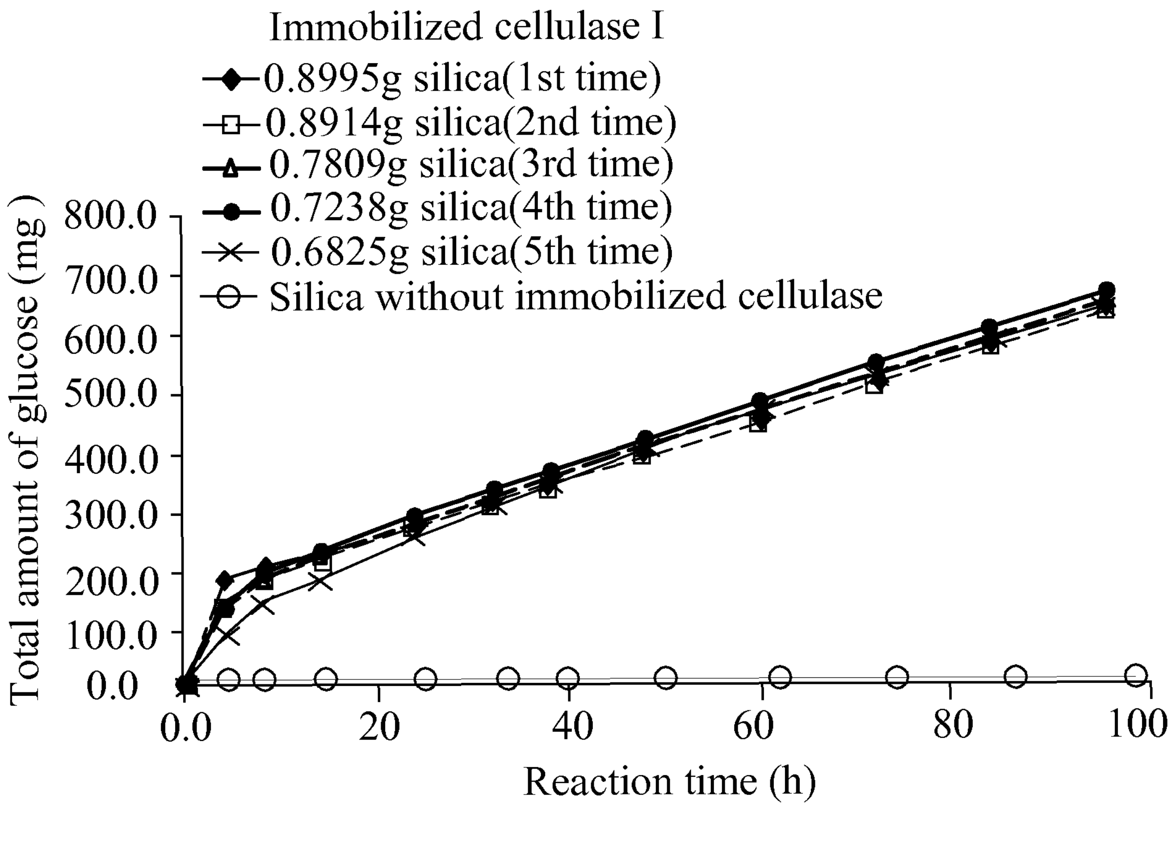
Immobilized cellulase I
0.8995g silica(1st time)
0.8914g silica(2nd time)
0.7809g silica(3rd time)
0.7238g silica(4th time)
0.6825g silica(5th time)
Silica without immobilized cellulase
Total amount of glucose (mg)
800.0
700.0
600.0
500.0
400.0
300.0
200.0
100.0
0.0
0.0
20
40
60
80
100
Reaction time (h)

FIG. 3D

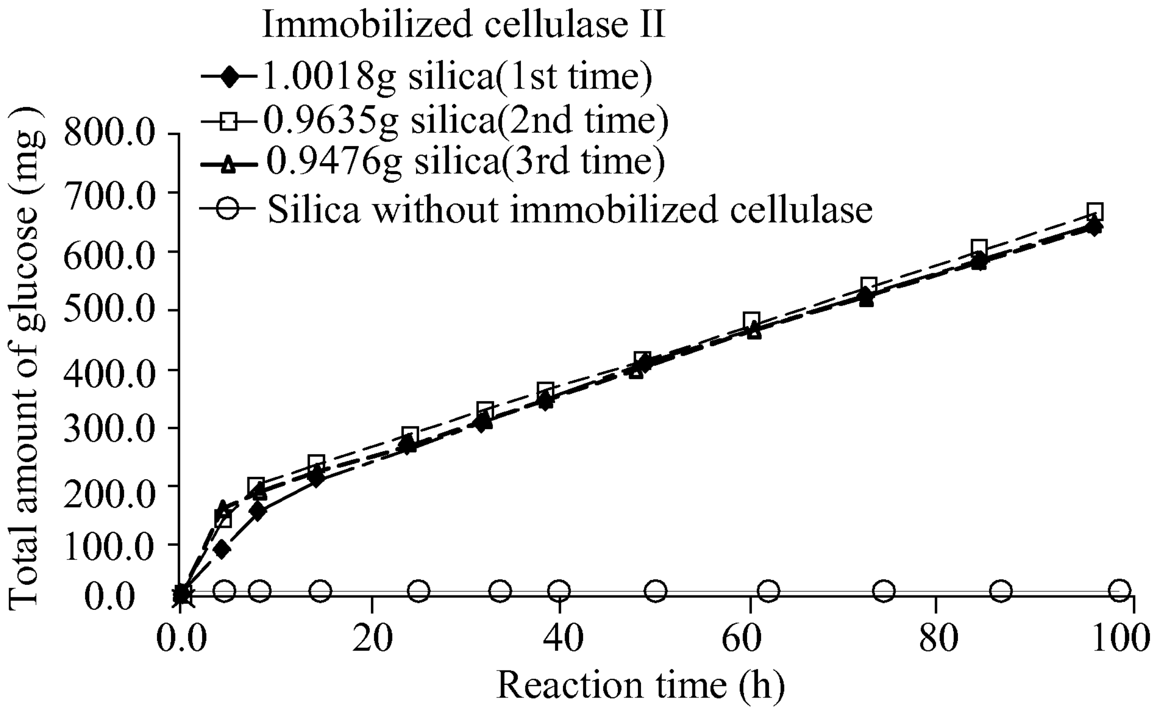
Immobilized cellulase II
1.0018g silica(1st time)
0.9635g silica(2nd time)
0.9476g silica(3rd time)
Silica without immobilized cellulase
Total amount of glucose (mg)
800.0
700.0
600.0
500.0
400.0
300.0
200.0
100.0
0.0
0.0
20
40
60
80
100
Reaction time (h)

FIG. 3E

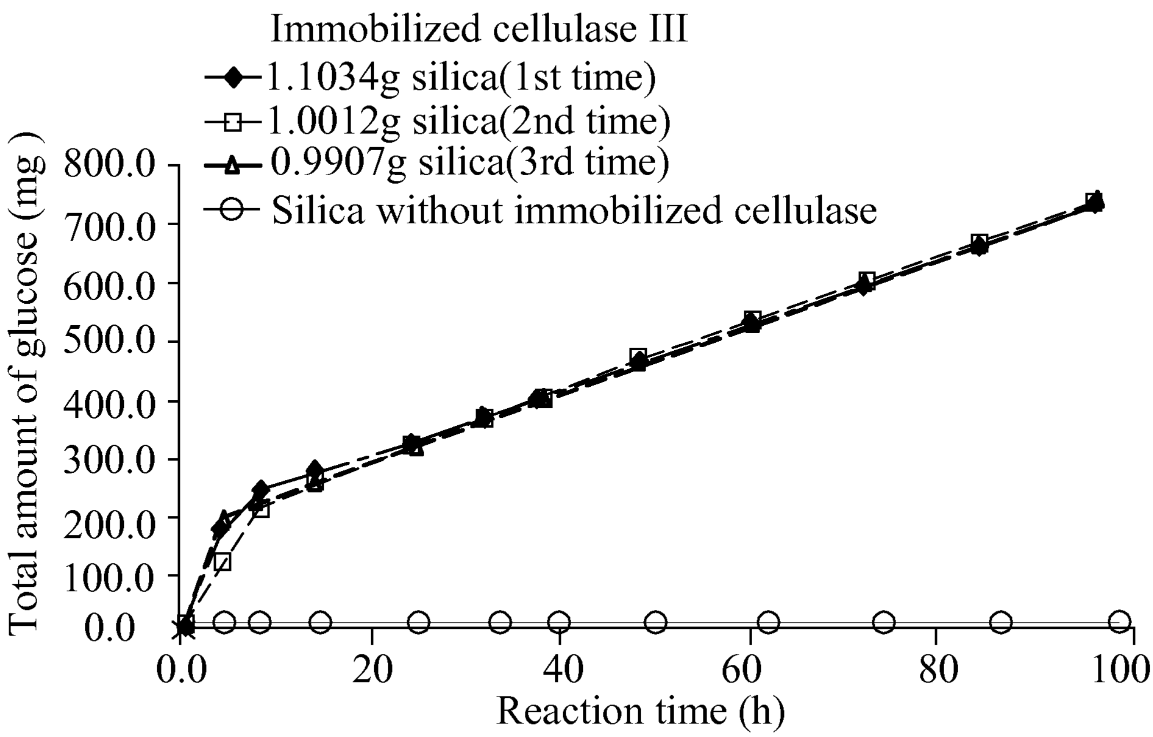
Immobilized cellulase III
1.1034g silica(1st time)
1.0012g silica(2nd time)
0.9907g silica(3rd time)
Silica without immobilized cellulase
Total amount of glucose (mg)
800.0
700.0
600.0
500.0
400.0
300.0
200.0
100.0
0.0
0.0
20
40
60
80
100
Reaction time (h)

FIG. 3F

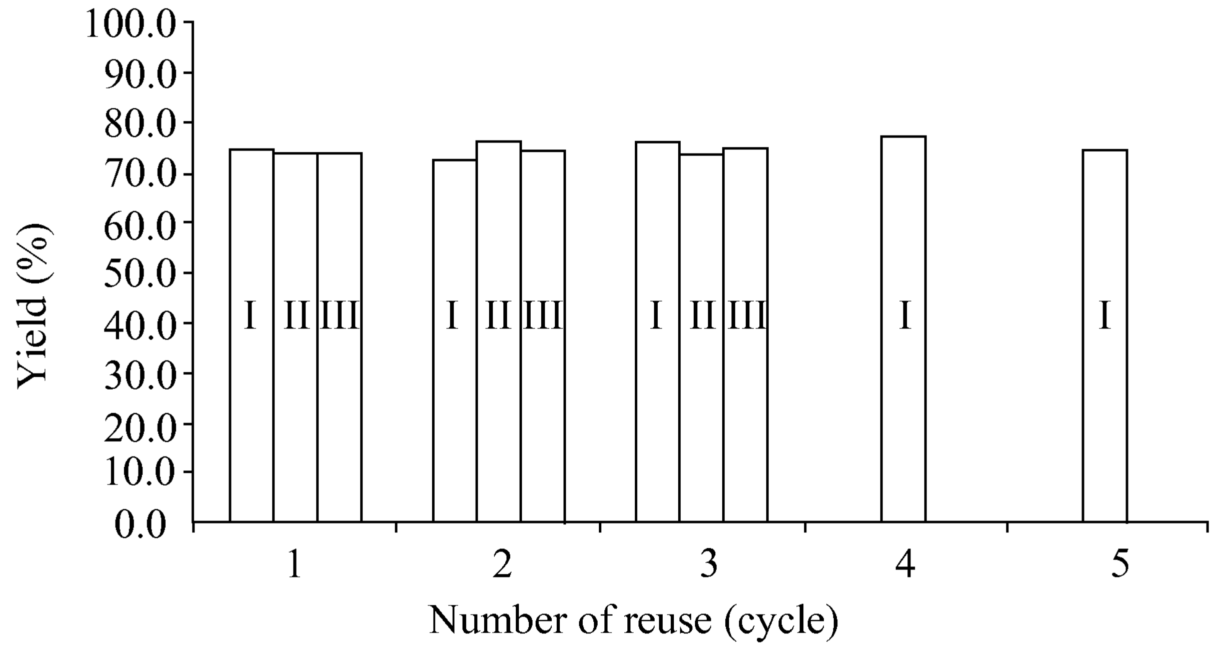
100.0
90.0
80.0
70.0
60.0
50.0
40.0
30.0
20.0
10.0
0.0
Yield (%)
I
II
III
1
2
3
4
5
Number of reuse (cycle)

FIG. 4A

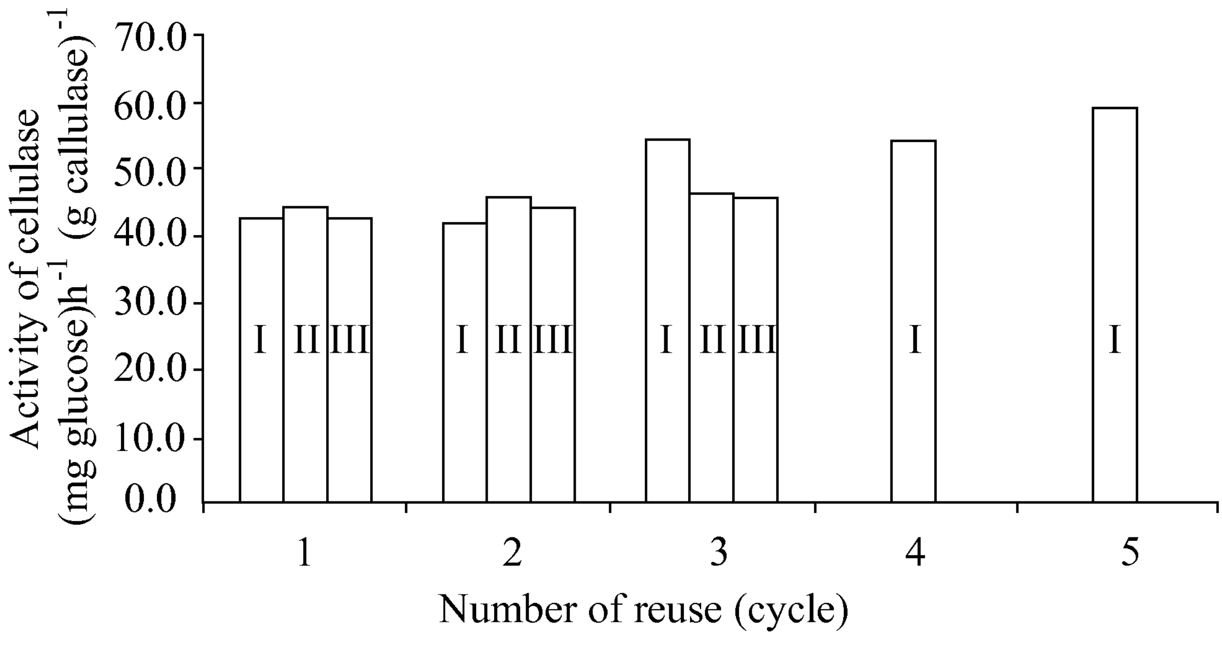
70.0
60.0
50.0
40.0
30.0
20.0
10.0
0.0
Activity of cellulase
(mg glucose)h-1 (g callulase)-1
I
II
III
1
2
3
4
5
Number of reuse (cycle)

FIG. 4B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BROWN, K.R. ; WALTER, D.G. ; NATAN, M.J.** Seeding of colloidal Au nanoparticle solutions. 2. improved control of particle size and shape. *Chem. Mater.,* 2000, vol. 12, 306-313
- **CHAKRABARTI, A. C. ; STOREY, K. B.** Immobilization of cellulase using polyurethane foam. *Appl. Biochem. Biotechnol.,* 1988, vol. 19, 189-207
- **CHANG, R. H.-Y. ; JANG, J. ; WU, K. C.-W.** Cellulase immobilized mesoporous silica nanocatalysts for efficient cellulose-to-glucose conversion. *Green Chem.,* 2011, vol. 13, 2844-2850
- **CHENG, C.** Cellulase activity in different buffering media during waste paper hydrolysis by HPLC. *J. Chin. Chem. Soc.,* 1998, vol. 45, 679-688
- **CHENG, C. ; CHANG, K.-C.** Sampling, dilution, and loading device-coupled high-performance liquid chromatography method for successive on-line analyses of major carbohydrate products in immobilized cellulase hydrolysate of paper cellulose. *Anal. Sci.,* 2007, vol. 23, 305-310
- **CHENG, C. ; CHANG, K.-C. ; CHEN, C.-S. ; PIJANOWSKA, D. G.** Biosensor with nano-gold particle modified pencil lead carbon electrode for long-term glucose monitoring of waste tree branch hydrolysis. *J. Chin. Chem. Soc.,* 2011, vol. 58, 1-10
- **CHENG, C. ; CHANG, K.-C. ; PIJANOWSKA, D. G.** On-line flow injection analysis using gold particle modified carbon electrode amperometric detection for real-time determination of glucose in immobilized enzyme hydrolysate of waste bamboo chopsticks. *J. Electroanal. Chem.,* 2012, vol. 666, 32-41
- **CHENG, C. ; CHEN, C.-S. ; HSIEH, P.-H.** On-line desalting and carbohydrate analysis for immobilized enzyme hydrolysis of waste cellulosic biomass by column-switching high-performance liquid chromatography. *J. Chromatogr.,* 2010, vol. A (1217), 2104-2110
- **CHO, E. J. ; JUNG, S. ; KIM, H. J. ; LEE, Y. G. ; NAM, K. C. ; LEE, H.-J. ; BAE, H.-J.** Co-immobilization of three cellulases on Au-doped magnetic silica nanoparticles for the degradation of cellulose. *Chem. Commun.,* 2012, vol. 48, 886-888
- **GE, Y. ; BURMAA, B. ; ZHANG, S. ; WANG, S. ; ZHOU, H. ; LI, W.** Co-immobilization of cellulase and glucose isomerase by molecular deposition technique. *Biotechnol. Tech.,* 1997, vol. 11, 359-361
- **GEDDES, C.C. ; PETERSON, J.J. ; ROSLANDER, C. ; ZACCHI, G. ; MULLINIX, M.T. ; SHANMUGAM, K.T. ; INGRAM, L.O.** Optimizing the saccharification of sugar cane bagasse using dilute phosphoric acid followed by fungal cellulases. *Bioresour. Technol.,* 2010, vol. 101, 1851-1857
- **GUPTA, P. ; KUMAR, V. ; PAUL, S., J.** Silica functionalized sulfonic acid catalyzed one-pot synthesis of 4,5,8a-Triarylhex-ahydropyrimido [4,5-d]pyrimidine-2,7(1H,3H)-diones under liquid phase catalysis. *Braz. Chem. Soc.,* 2010, vol. 21, 349-354
- **HARTONO, S. B. ; QIAO, S. Z. ; LIU, J. ; JACK, K. ; LADEWIG, B. P. ; HAO, Z. ; LU, G. Q. M.** Functionalized mesoporous silica with very large pores for cellulase immobilization. *J. Phys. Chem. C.,* 2010, vol. 114, 8353-8362
- **HUNG, T.-C. ; FU, C.-C. ; SU, C.-H. ; CHEN, J.-Y. ; WU, W.-T. ; LIN, Y.-S.** Immobilization of cellulase onto electrospun polyacrylonitrile (PAN) nanofibrous membranes and its application to the reducing sugar production from microalgae. *Eznyme Microb. Tech.,* 2011, vol. 49, 30-37
- **JAIN, P. ; WILKINS, E.S.** Cellulase immobilized on modified nylon for saccharification of cellulose. *Biotechnol. Bioeng.,* 1987, vol. 30, 1057-1062
- **JONES, P. O. ; VASUDEVAN, P. T.** Cellulose hydrolysis by immobilized Trichoderma reesei cellulase. *Biotechnol. Lett.,* 2010, vol. 32, 103-106
- **KANNAN, K. R. ; JASRA, V.** Improved catalytic hydrolysis of carboxy methyl cellulose using cellulase immobilized on functionalized meso cellular foam. *J. Porous Mat.,* 2011, vol. 18, 409-416
- **LI, C. ; YOSHIMOTO, M. ; FUKUNAGA, K. ; NAKAO, K.** Characterization and immobilization of liposome-bound cellulase for hydrolysis of insoluble cellulose. *Bioresour. Technol.,* 2007, vol. 98, 1366-1372
- **NAKAMURA, Y. ; SAWADA, T. ; INOUE, E.** Enhanced ethanol production from enzymatically treated steam-exploded rice straw using extractive fermentation. *J. Chem. Technol. Biotechnol.,* 2001, vol. 76, 879-884
- **QI, B.-K. ; CHEN, X.-R. ; SHEN, F. ; SU, Y. ; WAN, Y.-H.** Optimization of enzymatic hydrolysis of wheat straw pretreated by alkaline peroxide using response surface methodology. *Ind. Eng. Chem. Res.,* 2009, vol. 48, 7346-7353

- **SHAFIZADEH, F. ; BRADBURY, A. G. W.** Thermal degradation of cellulose in air and nitrogen at low temperatures. *J. Appl. Polym. Sci.,* 1979, vol. 23, 1431-1442
- **SHIMOKAWA, T. ; ISHIDA, M. ; YOSHIDA, S. ; NOJIRI, M.** Effects of growth stage on enzymatic saccharification and simultaneous saccharification and fermentation of bamboo shoots for bioethanol production. *Bioresour. Technol.,* 2009, vol. 100, 6651-6654
- **TÉBÉKA, I. R. M. ; SILVA, A. G. L. ; PETRI, D. F. S.** Hydrolytic activity of free and immobilized cellulose. *Langmuir,* 2009, vol. 25, 1582-1587
- **VAILLANT, F. ; MILLAN, A. ; MILLAN, P. ; DORNIER, M. ; DECLOUX, M. ; REYNES, M.** Co-immobilized pectinlyase and endocellulase on chitin and Nylon supports. *Process Biochem.,* 2000, vol. 35, 989-996
- **WU, C.-C. ; CHENG, C.** A study of the hydrolysis of waste paper cellulose with a vertically hanging immobilized cellulase reactor and the reuse of the immobilized cellulose. *J. Chin. Chem. Soc.,* 2005, vol. 52, 85-95
- **WU, L. ; YUAN, X. ; SHEN, J.** Immobilization of cellulase in nanofibrous PVA membranes by electrospinning. *J. Membr. Sci.,* 2005, vol. 250, 167-173